# FASCICULE DE BREVET EUROPEEN

(11) **EP 1 638 617 B1**
(45) Date de publication et mention de la délivrance du brevet: **19.09.2012**
(21) Numéro de dépôt: 04767486.6
(22) Date de dépôt: 28.06.2004
(51) Int. Cl.: A61L 2/14

(54) **DISPOSITIF DE STERILISATION PAR PLASMA POST-DECHARGE**
VORRICHTUNG ZUR STERILISATION DURCH PLASMA-NACHENTLADUNG
POST-DISCHARGE PLASMA STERILISATION DEVICE

(30) Priorité: 27.06.2003 FR 0307799
(43) Date de publication de la demande: 29.03.2006
(73) Titulaire: Satelec-Société pour la conception des applications des Techniques Electroniques, F-33700 Merignac (FR)
(72) Inventeur: DIERAS, Francis, F-33000 Bordeaux (FR); RICARD, André, F-31400 Toulouse (FR); SIXOU, Michel, F-31130 Balma (FR); VILLEGER, Sandrine, F-31062 Toulouse (FR)
(74) Mandataire: Desormiere, Pierre-Louis
(86) Numéro de dépôt international: PCT/FR2004/001640
(87) Numéro de publication internationale: WO 2005/000366

(56) Documents cités:
- EP-A- 0 377 799
- WO-A-03/043666
- WO-A-2004/050128
- WO-A1-2004/016291
- DE-A- 19 715 583
- FR-A- 2 759 590
- FR-A- 2 790 962
- FR-A1- 2 814 079
- FR-A1- 2 821 557
- US-A- 3 948 601

## Description

La présente invention concerne un dispositif de stérilisation notamment destiné à des instruments ou des appareils médicaux ou chirurgicaux.

Dans les milieux médicaux, la stérilisation est habituellement obtenue au moyen d'un autoclave dans lequel les instruments à stériliser sont portés à une température élevée déterminée, de l'ordre de 120°C, et ceci pendant des périodes de temps déterminées avec des cycles imposés par la législation. On notera tout d'abord que les autoclaves sont limités à la stérilisation d'objets de faible volume, ce qui en exclut l'utilisation pour assurer la stérilisation, par exemple, de conduites d'appareils tels que des dialyseurs ou des unités de traitement dentaires. Par ailleurs l'application d'une température supérieure à 100°C aux instruments et accessoires chirurgicaux modernes, crée de nombreuses contraintes et empêche notamment de soumettre à stérilisation les objets ou les accessoires fragiles comportant par exemple des parties en matériaux polymères de synthèse, qui sont habituellement particulièrement thermosensibles.

C'est pourquoi on s'est tourné, ces dernières années, vers des procédés permettant de réaliser des stérilisations à basse température.

Parmi ces procédés, on retiendra tout particulièrement ceux faisant appel au plasma gazeux. On rappellera que dans ces techniques on utilise un gaz, n'ayant pas de lui-même des propriétés bactéricides que l'on soumet à un champ électrique dont l'intensité est suffisamment élevée pour provoquer son ionisation et la dissociation de ses molécules, si bien que l'on obtient ainsi un plasma qui est constitué d'ions et d'électrons. On a constaté que le plasma possédait des propriétés bactéricides élevées qui ont été utilisées pour assurer la stérilisation d'instruments médicaux et chirurgicaux. A cet effet, le plasma ainsi produit est admis dans une chambre de traitement où il est mis en présence des instruments que l'on souhaite stériliser.

On sait cependant que si le plasma possède des propriétés stérilisantes élevées, il présente l'inconvénient de posséder également un effet destructeur sur certaines matières telles que notamment les matières plastiques de synthèse, ce qui exclut son utilisation pour la stérilisation de nombreux instruments médicaux ou chirurgicaux.

On sait également que le gaz produit en aval du plasma, désigné ci-après "gaz de post-décharge", possède des propriétés stérilisantes. Ce gaz qui est généré à la fin du plasma n'est plus soumis à l'effet du champ électrique, si bien que les électrons et les ions qui constituent le plasma disparaissent par recombinaison dans le gaz et après diffusion sur les parois du tube.

On a ainsi proposé dans le brevet WO 00/72889, un procédé de stérilisation qui fait notamment appel, en tant que gaz constitutif du plasma, à un mélange d'oxygène et d'azote. Suivant cette technique on a constaté que la présence d'oxygène atomique dans le gaz de post-décharge a pour effet de soumettre à une action d'oxydation les polymères utilisés dans le domaine chirurgical, qu'il s'agisse de parties d'instruments tels que notamment des pièces à main dentaires, des appareils à ultrasons, des endoscopes, des cathéters, des joints, des moteurs ou des appareils divers.

De plus, lors de la formation du plasma gazeux, l'interaction de l'oxygène atomique et de l'azote atomique a pour effet de produire un rayonnement ultraviolet dont l'action bactéricide s'ajoute à l'effet du gaz de post-décharge lui-même. Cette fonction de stérilisation produite par l'ultraviolet si elle est intéressante en ce qu'elle améliore la puissance de stérilisation du dispositif, présente cependant un grave inconvénient en ce que les effets des rayons ultraviolets viennent encore ajouter au caractère d'agressivité du traitement.

Les documents FR 2 759 590, DE 197 15 583 et EP 0 377 799 divulguent un dispositif de stérilisation d'objets comprenant des moyens aptes à créer un plasma gazeux à partir d'un flux gazeux, des moyens aptes à mettre en contact lesdits objects avec un flux post-décharge issu du plasma dans une chambre de stérilisation pourvue de moyens de chauffage.

Le document US 3 948 601 décrit un dispositif de stérilisation d'objects utilisant un flux post-décharge issu d'un plasma gazeux.

La présente invention a pour but de pallier les inconvénients précités en proposant un dispositif de stérilisation à basse température faisant appel à un plasma permettant d'éviter toute émission d'oxygène et de rayons ultraviolets en cours de traitement, ce qui permet de respecter l'intégrité des appareils et accessoires à stériliser comportant des matières sensibles aux phénomènes d'oxydation ainsi qu'aux ultraviolets, et ceci sans diminuer pour autant l'efficacité du dispositif.

La présente invention a ainsi pour objet un dispositif de stérilisation d'objets, notamment d'instruments médicaux ou chirurgicaux, comme défini dans la revendication 1.

Le flux de post-décharge qui est issu du plasma gazeux est admis dans une chambre de stérilisation dans laquelle sont disposés lesdits objets. Les parois de cette chambre de stérilisation sont constituées d'un matériau possédant une faible capacité de recombinaison des atomes d'azote, à savoir du verre et/ou de la céramique et/ou un polymère. Les objets à stériliser sont disposés sur un porte-objet métallique dont la nature sera telle que, sous l'effet de la recombinaison des atomes d'azote, ce porte-objet s'échauffe et assure le réchauffage des objets qu'il contient. Ce porte-objet, réalisé en laiton, pourra également être pourvu de ses propres moyens de chauffage.

Le champ électrique sera de préférence produit par un générateur de micro-ondes, mais il pourrait l'être également par des décharges à courant continu ou pulsé ou par des radiofréquences.

Dans un mode de mise en oeuvre de l'invention la chambre de stérilisation pourra être constituée d'un autoclave et cet autoclave pourra constituer les moyens de chauffage des instruments à stériliser.

Par ailleurs les moyens propres à générer le plasma pourront être contenus dans la porte de l'autoclave.

Dans une variante de mise en oeuvre de l'invention le chauffage des objets contenus dans la chambre de stérilisation pourra également être assuré en dotant les parois de la chambre de stérilisation de moyens de chauffage additionnels, notamment électriques.

La présente invention est particulièrement intéressante en ce qu'elle permet d'assurer la stérilisation des conduits et des cavités internes d'appareils et même d'appareils de fort volume tels que par exemple des unités de traitement dentaire, des appareils de dialyse etc...

On pourra amener le flux de post-décharge à la fois dans la chambre de traitement et dans l'appareil par un orifice de celui-ci et l'extraire, par exemple par aspiration, à la fois de la chambre de traitement et de l'appareil par un second orifice.

On pourra suivant l'invention élever, au cours du traitement, la température des instruments, cette augmentation de température pouvant être obtenue par chauffage du porte-objets, ou par chauffage de la chambre de stérilisation, mais également par recombinaison des atomes du gaz de post-décharge sur les surfaces du porte-objets.

En effet on sait que les atomes d'azote, produits par la post-décharge d'azote pur, réagissent entre eux, par recombinaison atomiques à la surface des objets à traiter et que ces réactions sont exothermiques. Ainsi, il a été établi que, dans les conditions expérimentales testées, à savoir : une pression de 665Pa, une puissance de générateur de micro-ondes de 100W et un débit de 1 1/min, la température de surface des matériaux atteignait 80°C pour le laiton, 55°C pour l'acier, 60°C pour l'aluminium, 55°C pour le titane, 40°C pour la céramique et 37°C pour le verre.

Or on a établi, dans le cas de la bactérie Escherichia Coli qu'une température de 60°C était nécessaire pour induire une décroissance de la population bactérienne de 10⁶ en 40 minutes d'exposition à la post-décharge d'azote. Ainsi, afin d'assurer une stérilisation efficace des instruments quelque soit leur nature, il est nécessaire de porter leur surface, à une température minimale de 60°C, au cours de la stérilisation.

Par ailleurs, suivant l'invention, en faisant appel à un flux de gaz constitué exclusivement d'azote, on évite la formation, lors de la production du plasma, de rayons ultraviolets qui ont pour effet de porter atteinte à l'intégrité des matières de synthèse utilisées la plupart du temps dans les instruments ou accessoires chirurgicaux.

On décrira ci-après, à titre d'exemple non limitatif, une forme d'exécution de la présente invention, en référence au dessin annexé sur lequel :
- La figure 1 est une vue schématique d'un dispositif de stérilisation suivant l'invention.
- La figure 2 est une variante de mise en oeuvre du dispositif de stérilisation représenté sur la figure 1.
- La figure 3 est une vue schématique d'une variante de mise en oeuvre du dispositif suivant l'invention.
- La figure 4 est un schéma représentant la décroissance bactérienne de E.Coli en fonction du temps de stérilisation, et ceci pour différentes valeurs de température de chauffage d'un porte-instrument.
- Les figures 5 et 6 sont des vues schématiques de deux applications à la stérilisation des conduits et cavités internes d'un endoscope et d'un fibroscope, qui ne sont pas conformes à l'invention.
- La figure 7 est une vue schématique d'un exemple de stérilisation de la surface externe et des conduits et cavités internes d'un appareil.

La figure 8 est une vue schématique d'une application du dispositif suivant l'invention à la stérilisation des conduits et cavités internes d'un appareil de dialyse.

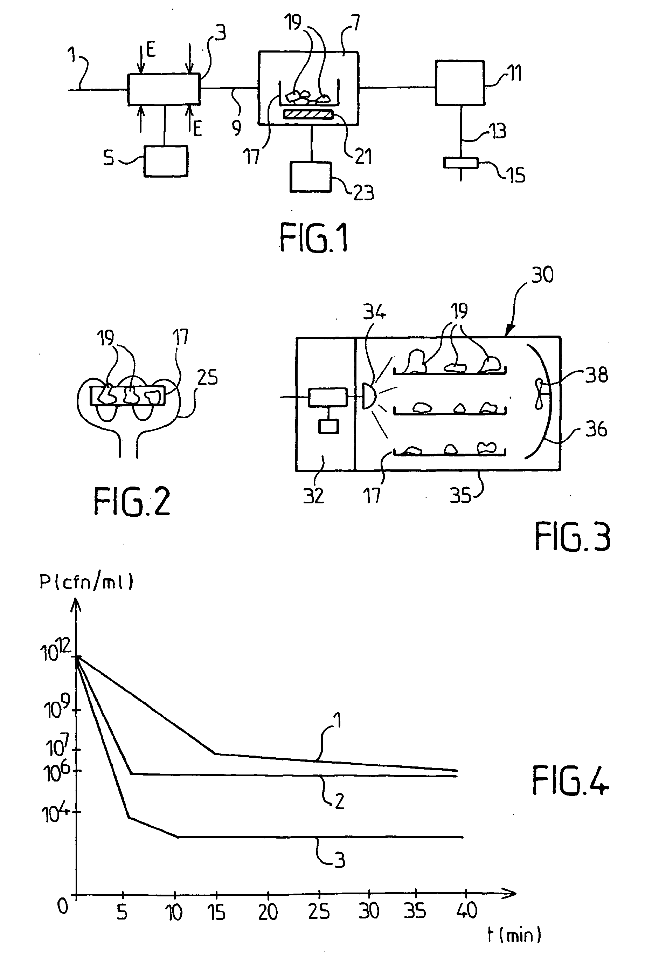

On a représenté sur la figure 1, de façon très schématique, un dispositif de stérilisation à plasma gazeux suivant l'invention. Ce dispositif comprend une conduite d'arrivée 1 d'un flux d'azote qui traverse une enceinte sous vide soumise à l'action d'un générateur de champ électrique constitué par un générateur 3 de micro-ondes à 2,45 GHz, dont la puissance est régulée par des moyens de contrôle 5. Le gaz de post-décharge généré par le plasma ainsi produit (de façon connue), est amené dans une chambre de traitement 7 par une conduite 9. Cette chambre de traitement 7 est disposée dans la zone de post-décharge du plasma et se trouve en communication avec une pompe à vide 11. Cette dernière entraîne le gaz de post-décharge dans la chambre de traitement 7 et assure l'évacuation des gaz vers l'extérieur par une conduite 13 pourvu des filtres appropriés 15.

La chambre de traitement 7 comporte un porte-objet métallique 17 qui est destiné à recevoir les objets 19 que l'on souhaite stériliser.

Le porte-objet 17 est pourvu de moyens de chauffage 21 dont la température est contrôlée par un dispositif de commande 23. Ces moyens de chauffage peuvent notamment être constitués d'une résistance électrique ou, ainsi que représenté sur la figure 2 par des moyens de chauffage par induction 25.

Ainsi que représenté sur la figure 3 la chambre de traitement peut être constituée d'un autoclave du type de ceux qui sont utilisés pour assurer la stérilisation des instruments médicaux, ou chirurgicaux.

Sur cette figure l'autoclave 30 est ainsi constitué d'une enceinte 35, de forme sensiblement parallélépipédique qui est fermée sur l'un de ses côtés par une porte pivotante 32. Cette porte pivotante est suffisamment épaisse pour renfermer les divers éléments nécessaires à la génération du plasma. Elle comporte, sur sa face frontale, une buse 34 de sortie du gaz de post-décharge destinée à alimenter l'intérieur de l'enceinte. Cette buse 34 pourra avantageusement se terminer par un ou plusieurs injecteurs permettant notamment d'homogénéiser le flux du gaz de post-décharge.

Dans le mode de mise en oeuvre représenté sur la figure 3 l'enceinte 35 est pourvue sur sa paroi opposée à la porte 32 d'un "réflecteur" 36 et d'un ventilateur 38 qui contribue à l'homogénéisation du gaz de post-décharge dans l'enceinte 35. Une telle disposition est intéressante en ce qu'elle permet à l'utilisateur de disposer d'un autoclave à plusieurs fonctions, à savoir une fonction classique d'autoclave et une fonction dans laquelle on stérilise par gaz de post-décharge et à basse température. Ainsi, en fonction des objets à stériliser, l'utilisateur aura la possibilité de faire appel au mode de stérilisation le plus approprié.

Dans cette variante de mise en oeuvre de l'invention l'autoclave pourra être utilisé pour porter à la température souhaitée les objets à stériliser.

Il a en effet été constaté que l'on pouvait obtenir un gaz de post-décharge possédant des propriétés bactéricides à partir d'un flux gazeux d'alimentation constitué exclusivement d'azote sans faire appel pour autant à de l'oxygène atomique ainsi que l'enseigne l'état antérieur de la technique.

On a établi qu'un gaz de post-décharge obtenu à partir d'un flux gazeux constitué exclusivement d'azote avait un effet biocide marqué sur les bactéries.

On a également constaté que l'importance de l'effet biocide obtenu était lié à la nature du porte-objet utilisé et à la température à laquelle on portait celui-ci au cours de l'opération de stérilisation.

On a ainsi, dans un porte-instrument en acier comportant des moyens de chauffage électrique du type de celui représenté sur la figure 1, disposé une population bactérienne Escherichia Coli que l'on a soumis à l'action d'un gaz de post-décharge obtenu à partir d'un flux d'azote pur sous une pression de 6hPa.

On a chauffé le porte-instrument à des températures de 60°C, 80°C et 120°C et l'on a mesuré la population bactérienne restante au bout respectivement de cinq, dix, quinze et quarante minutes. Les courbes correspondantes de la figure 4 représentent la variation de colonies de bactéries par ml en fonction du temps.

On retiendra notamment de celle-ci les résultats suivants :

| Courbe n° | T° porte-instrument (°C) | Durée | Coefficient de diminution |
|---|---|---|---|
| 1 | 60 | 15 min | 10⁵ |
| | 60 | 40 min | 10⁶ |
| 2 | 80 | 5 min | 10⁶ |
| 3 | 120 | 5 min | 10⁸ |

On constate ainsi que la présente invention permet, en fonction du niveau de température qu'il est possible d'appliquer à un objet à stériliser sans le dégrader, de sélectionner le mode de stérilisation qui lui est applicable. Ainsi, si l'objet considéré est en mesure de supporter une température de 120°C on pourra le soumettre à un traitement particulièrement rapide, puisque celui-ci ne durera que 5 min, en chauffant le porte-objet à 120°C. On aura alors une diminution de la population bactérienne de 10⁸.

Pour un objet plus fragile, qui ne peut supporter des températures supérieures à 80°C, on chauffera le porte-objet à cette température et le traitement durera alors également 5 min, le coefficient de diminution de la population bactérienne étant alors de 10⁶. Mais on sait qu'en matière de stérilisation d'instruments médicaux ou chirurgicaux un temps de stérilisation de 40 min est tout à fait acceptable en regard des techniques classiques connues, et les mesures précédentes montrent qu'au bout de ce temps on obtient une diminution de la population bactérienne de 10⁶ à une température de 60°C ce qui est particulièrement performant.

Enfin, dans les cas où une diminution de la population bactérienne de 10⁵ est suffisante et où l'on souhaite mettre en oeuvre un temps moins long, si l'objet à stériliser est particulièrement fragile on chauffera le porte-objet à une température de 60°C et on appliquera un temps de traitement de 15 min.

La présente invention permet également d'assurer la stérilisation d'appareils qui, en raison de leur nature, ou de leurs dimensions, ne sont pas stérilisables dans les stérilisateurs de type classique.

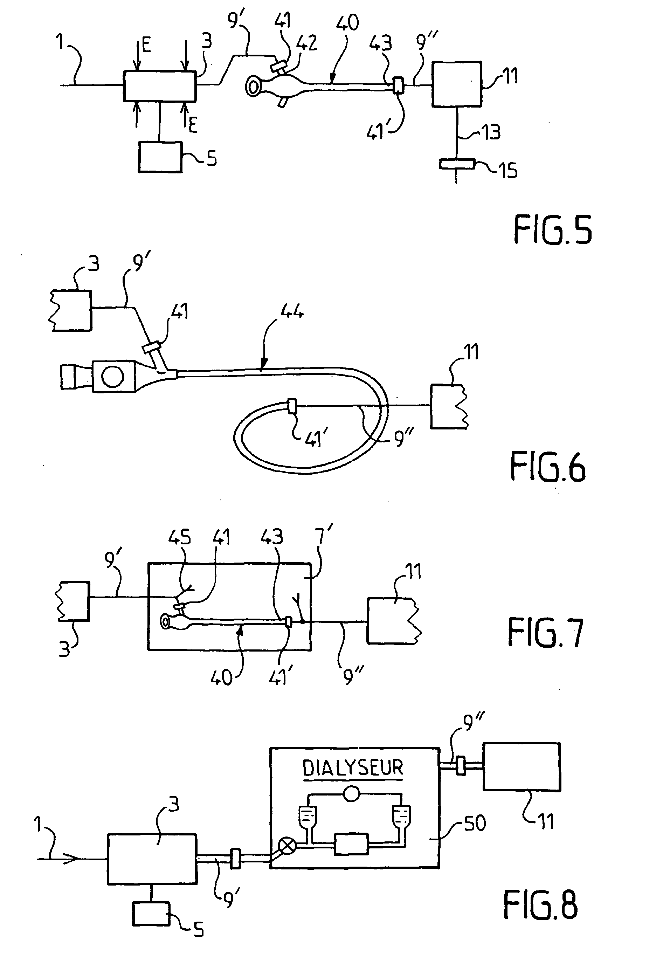

Ainsi que représenté sur la figure 5, ne représentant pas l'invention, on a ainsi appliqué le dispositif de stérilisation représenté sur la figure 1 à la stérilisation d'un endoscope 40. A cet effet une entrée 42 de celui-ci est reliée au moyen d'un connecteur 41 à une conduite 9' reliée à la sortie du générateur 3 de plasma, de façon que le gaz post-décharge se forme à l'intérieur d'une chambre de stérilisation constituée par les conduites et cavités internes de l'endoscope 40. La sortie 43 de celui-ci est de même reliée, par l'intermédiaire d'un connecteur 41', à une conduite 9" reliée à une pompe à vide 11. Suivant l'invention le gaz post-décharge qui traverse l'intérieur des cavités de l'endoscope assurera la stérilisation de celui-ci.

On notera qu'un tel mode d'utilisation est particulièrement intéressant d'une part en ce qui concerne sa facilité de sa mise en oeuvre par le praticien et, d'autre part, en ce qu'il permet d'assurer la stérilisation d'appareils qui peuvent comporter sur leur surface externe des parties réalisées dans des matières ne pouvant résister aux températures exigées par les stérilisations de type classique.

Ainsi que représenté sur la figure 6, ne représentant pas l'invention, on pourra également appliquer un dispositif de stérilisation identique à d'autres appareils et notamment à un fibroscope 44.

On pourra bien entendu suivant l'invention assurer également la stérilisation de l'ensemble de l'appareil, c'est-à-dire de ses conduites et cavités internes ainsi que de sa surface externe, lorsque cela est souhaité, en disposant celui-ci à l'intérieur d'une chambre de stérilisation 7' qui est en communication par une conduite 9' avec le générateur 3 de plasma, cette conduite étant reliée à une entrée de l'endoscope par un connecteur 41 et étant également en communication par une buse 45 avec l'intérieur de la chambre de stérilisaion 7' dans laquelle se forme le gaz de post-décharge, la sortie 43 de l'endoscope 40 ainsi que le volume interne de la chambre de stérilisation étant reliés à une pompe à vide 11 ainsi que représenté sur la figure 7.

On peut également utiliser le dispositif suivant l'invention pour assurer la stérilisation des conduites et volumes internes d'une unité de traitement dentaire en reliant, une entrée des canalisations de cette unité à l'arrivée du gaz de post-décharge, et la sortie de celle-ci à une pompe à vide.

Une autre application particulièrement intéressante de l'invention consiste en la stérilisation d'appareils de dialyse, ainsi que représenté sur la figure 8. L'appareil de dialyse 50 est ainsi relié par son entrée à une conduite d'arrivée 9' du gaz de post-décharge et sa sortie est reliée à une pompe à vide 11.

## Revendications

1. Dispositif de stérilisation d'objets (19,40,42,50), notamment d'instruments médicaux ou chirurgicaux, du type apte à créer à partir d'un flux gazeux soumis à un champ électrique un plasma gazeux dont le flux de post-décharge qui en est issu est mis en contact avec la surface des objets à traiter, comprenant :
- une chambre de stérilisation (7,7') dans laquelle sont disposés lesdits objets (19,40),
- des moyens aptes à produire ledit plasma à partir d'un flux gazeux exclusivement constitué d'azote,
- des moyens de chauffage desdits objets aptes à porter ces derniers, en cours de traitement, à une température d'au moins 60°C,
**caractérisé en ce que** les parois de la chambre de stérilisation sont constituées de verre et/ou de céramique et/ou d'un polymère possédant une faible capacité de recombinaison des atomes d'azote,
et **en ce que** les objets (19) sont disposés dans un porte-objet en laiton qui, sous l'effet de la recombinaison des atomes d'azote, s'échauffe et assure le réchauffage des objets (19) qu'il contient.

2. Dispositif suivant la revendication 1, **caractérisé en ce que** la chambre de stérilisation est constituée d'un autoclave.

3. Dispositif suivant la revendication 1, **caractérisé en ce que** le porte-objet comprend en outre des moyens de chauffage (21).

4. Dispositif suivant l'une des revendications 1 à 3, **caractérisé en ce que** les parois de la chambre de stérilisation (7) sont pourvues de moyens de chauffage additionnels notamment électriques.

5. Dispositif de stérilisation selon l'une des revendications précédentes, pour un objet de type appareil (40,42,50) comportant des conduits et cavités internes que l'on souhaite stériliser et des orifices d'entrée et de sortie, **caractérisé en ce qu'**il comporte des moyens d'injection du flux de post-décharge par un orifice d'entrée dudit appareil et des moyens de récupération du flux de post-décharge par un orifice de sortie.

## Claims

1. Device for sterilising objects (19, 40, 42, 50), in particular medical or surgical instruments, of the type adapted to create from a gaseous flow subjected to an electric field a gaseous plasma of which the post-discharge flow which issues therefrom is brought into contact with the surface of the objects to be treated, comprising:
. a sterilisation chamber (7, 7') in which said objects (19, 40) are disposed,
. means adapted to produce said plasma from a gaseous flow exclusively constituted by nitrogen,
. means for heating said objects adapted to take the latter, in the course of treatment, to a temperature of at least 60[deg.] C,
**characterized in that** the wall of the sterilisation chamber is constituted by glass and/or ceramics and/or a polymer presenting a low capacity of recombination of the nitrogen atoms,
and **in that** the objects (19) are arranged in a brass object-holder (17) which, under the effect of the recombination of the nitrogen atoms, heats up and ensures heating of the objects (19) that it contains.

2. Device according to claim 1, **characterized in that** the sterilisation chamber is constituted by an autoclave.

3. Device according to claim 7, **characterized in that** the object-holder further comprises heating means (21).

4. Device according to any one of claims 1 to 3, **characterized in that** the walls of the sterilisation chamber (7) are provided with additional, in particular electric, heating means.

5. Device for sterilising apparatus (40, 42, 50), in particular surgical ones, of the type adapted to create from a gaseous flow subjected to an electric field a gaseous plasma of which the post-discharge flow which issues therefrom is connected to the apparatus to be sterilized by means of a conduit, **characterized in that** it comprises:
. means adapted to produce said plasma from a gaseous flow exclusively constituted of nitrogen,
. means for injecting the post-discharge flow via an orifice in this apparatus through the tubes and internal cavities thereof, this flow being expelled through the other orifice.

## Patentansprüche

1. Verfahren zur Sterilisation von Gegenständen (19, 40, 42, 50), insbesondere von medizinischen oder chirurgischen Instrumenten, vom Typ, der geeignet ist, mit Hilfe eines einem elektrischen Feld ausgesetzten Gasstroms ein Gasplasma zu erzeugen, dessen Nachentladungsstrom, der hieraus hervorgegangen ist, mit der Oberfläche der zu behandelnden Gegenstände in Kontakt gebracht wird, umfassend:
- eine Sterilisationskammer (7, 7'), in der die Gegenstände (19, 40) angeordnet sind,
- Mittel, die geeignet sind, das Plasma mit Hilfe eines ausschließlich von Stickstoff gebildeten Gasstroms zu erzeugen,
- Mittel zum Erhitzen der Gegenstände, die geeignet sind, letztere während der Behandlung auf eine Temperatur von wenigstens 60 °C zu erhitzen,
**dadurch gekennzeichnet, daß** die Wände der Sterilisationskammer aus Glas und/oder aus Keramik und/oder aus einem Polymer mit einer geringen Fähigkeit zur Rekombination der Stickstoffatome bestehen,
und daß die Gegenstände (19) in einem Objektträger aus Messing angeordnet sind, der sich unter der Wirkung der Rekombination der Stickstoffatome erhitzt und das Erhitzen der Gegenstände (19), die er enthält, sicherstellt.

2. Vorrichtung nach Anspruch 1, **dadurch gekennzeichnet, daß** die Sterilisationskammer von einem Autoklaven gebildet ist.

3. Vorrichtung nach Anspruch 1, **dadurch gekennzeichnet, daß** der Objektträger ferner Heizmittel (21) umfaßt.

4. Vorrichtung nach einem der Ansprüche 1 bis 3, **dadurch gekennzeichnet, daß** die Wände der Sterilisationskammer (7) mit zusätzlichen Heizmitteln, insbesondere elektrischen, versehen sind.

5. Sterilisationsvorrichtung nach einem der vorhergehenden Ansprüche, für einen Gegenstand vom Typ Gerät (40, 42, 50), das Leitungen und Innenhohlräume, die sterilisiert werden sollen, sowie Einlaß- und Auslaßöffnungen umfaßt, **dadurch gekennzeichnet, daß** sie Mittel zum Einleiten des Nachentladungsstroms über eine Einlaßöffnung des Gerätes sowie Mittel zum Zurückgewinnen des Nachentladungsstroms über eine Auslaßöffnung umfaßt.
